# EUROPEAN PATENT APPLICATION

(11) **EP 3 438 869 A1**
(43) Date of publication of application: **06.02.2019**
(21) Application number: 17184469.9
(22) Date of filing: 02.08.2017
(51) Int. Cl.: G06F 21/62

(54) **ANONYMIZING DATA**

(71) Applicant: Koninklijke Philips N.V., 5656 AE Eindhoven (NL)
(72) Inventor: SAALBACH, Axel, 5656 AE Eindhoven (NL); BROSCH, Tom, 5656 AE Eindhoven (NL); BYSTROV, Daniel, 5656 AE Eindhoven (NL); BUSSA, Nagaraju, 5656 AE Eindhoven (NL); DINESH, Mysore Siddu, 5656 AE Eindhoven (NL)
(74) Representative: de Haan, Poul Erik

(57) **Abstract**

There is provided an apparatus (104) for storing medical imaging data. The apparatus comprises a processor configured to acquire a medical imaging study relating to a subject; identify elements in the medical imaging study that are indicative of the identity of the subject; anonymize the medical imaging study by removing the identified elements from the medical imaging study; deliver the anonymized medical imaging study for storage at a first location; and deliver data relating to the identified elements for storage at a second location. An apparatus for retrieving medical imaging data from storage, associated methods and a computer program product are also disclosed.

## Description

### FIELD OF THE INVENTION

The invention relates to medical imaging data and, more particularly, to anonymizing and storing medical imaging data and retrieving anonymized medical imaging data from storage.

### BACKGROUND OF THE INVENTION

Data storage can be costly when large amounts of data are generated, particularly if the data needs to be stored securely. Data generated in the field of medical imaging often contains confidential information and, therefore, should be stored in a secure environment. Typically, such medical imaging data is stored on a storage medium local to the system that generated the data, or on a storage medium shared between multiple medical institutions, so that the data can be accessed by medical professionals situated at different medical institutions.

To reduce the costs associated with the local storage of medical imaging data, it has been proposed to store the medical imaging data in a cloud-computing storage medium. Such cloud-based storage solutions may be less costly than storing data locally, but suffer from the problem that the confidentiality of patient or subject data may be compromised. For example, sensitive personal information, such as a subject's name and date of birth, may be made available to an unauthorized person. Also, if a series of scans of a subject are accessed (e.g. illegally) from a storage medium, it may be possible to construct an image of the subject from the scans.

One proposed method of improving the security of data stored in a cloud-based storage facility is to encrypt the data using known data encryption techniques. However, data encryption is not a secure long-term solution for sensitive confidential data, as advances in cryptographic research mean that decryption of data is becoming easier.

### SUMMARY OF THE INVENTION

It would be desirable to have a data storage means which addresses at least some of the afore-mentioned problems, and which enables data - particularly medical imaging data - to be stored and retrieved from storage in a secure manner. Due to the large costs involved with storing data on local data storage media, it would also be desirable to have an option to securely store data in a lower cost storage facility, such as a cloud-based storage facility. To better address one or more of these concerns, some aspects of the present invention provide a mechanism by which sensitive data may be stored in way which may reduce storage costs while maintaining confidentiality of the people to whom the data relates.

According to a first aspect, the invention provides an apparatus for storing medical imaging data. The apparatus comprises a processor configured to acquire a medical imaging study relating to a subject; identify elements in the medical imaging study that are indicative of the identity of the subject; anonymize the medical imaging study by removing the identified elements from the medical imaging study; deliver the anonymized medical imaging study for storage at a first location; and deliver data relating to the identified elements for storage at a second location.

By storing data which is considered to be sensitive data (i.e. the data relating to the identified elements) in a separate location to the less sensitive data (i.e. the anonymized study which does not contain subject-identifying data), the less sensitive data may be stored in a less secure and, therefore, lower cost storage facility. Thus, relatively higher cost secure storage may be used only for the sensitive data from a medical imaging study.

In some embodiments, the medical imaging study may comprise at least an image. The identified elements of the medical imaging study may comprise image features in the image which are indicative of the identity of the subject. Anonymizing the medical imaging study may comprise removing the identified image features from the image. The processor may be configured to deliver the anonymized image for storage as part of the anonymized medical imaging study at the first location; and deliver data relating to the identified image features for storage at the second location.

Since an image can be anonymized in a medical imaging study, large reductions in data storage requirements can be made. Large portions of an image may contain non-sensitive data, and this can be stored in a relatively lower cost storage facility. The sensitive data from the image, which may be a small proportion of the whole image, can be stored securely. Data storage costs can be greatly reduced in this way.

The processor may, in some embodiments, be configured to assign a first identifier to the anonymized medical imaging study; and assign a second identifier to the data relating to the identified elements. The first identifier and the second identifier may correspond to one another. By assigning identifiers to the stored portions, retrieval of the portions from storage can be achieved more efficiently, with a lower risk of incorrect data being retrieved.

In some embodiments, the processor is further configured to replace the removed identified image features with a mask in the anonymized medical imaging study. In this way, it may be possible to see the portion of the study from which any data has been removed.

Identifying image features in the image which are indicative of the identity of the subject may comprise applying at least one of: an atlas-based segmentation technique; a sliding-window detection technique; a generalized Hough transform voting technique; a regression forest voting technique; an image segmentation technique; and a deep-learning technique.

The image features in the image which are indicative of the identity of the subject may, in some embodiments, comprise at least one of: an anatomical feature, an annotation, an implant, and a tattoo. Such features can be recognized in an image, and known recognition techniques may be used to identify such features in a study, making the anonymization process more efficient.

In some embodiments, the processor may be configured to encrypt at least one of: data relating to the anonymized medical imaging study, and the data relating to the identified elements. By encrypting the data, the security of the stored data is further increased.

The medical imaging study may comprise at least non-image data. The processor may be configured to identify a data element in the non-image data that is indicative of the identity of the subject; anonymize the non-image data by removing the identified data element from the non-image data; deliver the anonymized non-image data for storage at the first location; and deliver the remaining non-image data for storage at the second location. By anonymizing the non-image data in the study, particularly in addition to anonymizing the image data in the study, the identity of the subject can be further protected.

Identifying a data element in the non-image data may, in some embodiments, comprise at least one of: identifying a data element in the non-image data that is present in a defined list of data elements known to be indicative of the identity of the subject; and identifying a data element in the non-image data using a text search engine.

According to a second aspect, the invention provides an apparatus for retrieving from storage medical imaging data associated with a subject. The apparatus comprises a processor configured to obtain, from a first storage location, an anonymized medical imaging study associated with the subject, the anonymized medical imaging study having had elements indicative of the identity of the subject removed therefrom; obtain, from a second storage location, data relating to the elements indicative of the identity of the subject; and form a medical imaging study by combining the anonymized medical imaging study and the data relating to the elements indicative of the identity of the subject.

In some embodiments, the second location may comprise a more secure storage medium than the first location. Thus, storage at the first location may be relatively lower cost than storage at the second, more secure, location.

The medical imaging study may comprise data formatted according to the Digital Imaging and Communications in Medicine (DICOM) standard. DICOM data includes DICOM data elements, so particular data entries (e.g. entries relating to sensitive data) can be easily identified and anonymized (e.g. removed).

According to a third aspect, the invention provides a method for storing medical imaging data, comprising acquiring a medical imaging study relating to a subject; identifying elements in the medical imaging study that are indicative of the identity of the subject; anonymizing the medical imaging study by removing the identified elements from the medical imaging study; delivering the anonymized medical imaging study for storage at a first location; and delivering data relating to the identified elements for storage at a second location.

According to a fourth aspect, the invention provides a method for retrieving from storage medical imaging data associated with a subject, the method comprising: obtaining, from a first storage location, an anonymized medical imaging study associated with the subject, the anonymized medical imaging study having had elements indicative of the identity of the subject removed therefrom; obtaining, from a second storage location, data relating to the elements indicative of the identity of the subject; and forming a medical imaging study by combining the anonymized medical imaging study and the data relating to the elements indicative of the identity of the subject.

According to a fifth aspect, the invention provides a computer program product comprising a non-transitory computer readable medium, the computer readable medium having computer readable code embodied therein, the computer readable code being configured such that, on execution by a suitable computer or processor, the computer or processor is caused to perform a method as described herein.

These and other aspects of the invention will be apparent from and elucidated with reference to the embodiments described hereinafter.

### BRIEF DESCRIPTION OF THE DRAWINGS

For a better understanding of embodiments of the invention, and to show more clearly how these may be carried into effect, reference will now be made, by way of example only, to the accompanying drawings, in which:
Figure 1 is a schematic illustration of an example of a system for storing medical imaging data;
Figure 2 is a schematic illustration of an example of an apparatus for storing medical imaging data according to an embodiment of the invention;
Figure 3 is an illustration of (a) scan images; and (b) anonymized scan images;
Figure 4 is a schematic illustration of an example of an apparatus for retrieving medical imaging data from storage according to an embodiment of the invention;
Figure 5 is a flowchart of an example of a method of storing medical imaging data according to an embodiment of the invention;
Figure 6 is a flowchart of an example of a method of retrieving medical imaging data from storage according to an embodiment of the invention; and
Figure 7 is an illustration of a computer-readable medium and a processor.

### DETAILED DESCRIPTION OF THE EMBODIMENTS

Data generated, for example, from a medical imaging procedure typically includes information from which a person to whom the data relates (e.g. a subject) may be identified (referred to as sensitive data, or subject-identifying data), along with data from which the person cannot be identified (referred to as less sensitive data). The information indicative of the identity of the subject, which may be referred to as sensitive data, may comprise protected health information (PHI) which, under US law, may comprise any information about health status, provision of health care, or payment for health care that can be linked to a specific individual.

For example, a medical imaging study may include a series of scan images acquired from a medical imaging procedure, such as a computed tomography (CT) scan or a magnetic resonance (MR) scan of a subject, along with information relating to the subject, such as the subject's name, date of birth and address, the medical facility at which the imaging data was acquired, parameters of the medical imaging system used to acquire the imaging data, and the like. The image, or scan, itself may also be considered confidential, particularly the subject's facial region, for example. The data included in the medical imaging study from which the subject could be identified may be a relatively small proportion of the data included in the study. However, secure storage of the data has typically required the entire medical imaging study to be stored securely, for example in a local secure storage medium, or by encrypting the data in the study and storing the encrypted data in a storage medium.

According to aspects of the present invention, sensitive data (i.e. data from which a subject may be identified) in a medical imaging study may be identified and removed from the medical imaging study so that a censored, or anonymized version of the medical imaging study may be stored in a relatively low-cost storage facility, such as a cloud-based storage facility. The sensitive data removed from the medical imaging study may be stored in a more secure storage facility, such as a local storage medium to which access is restricted.

Medical imaging data is often stored and exchanged according to a Digital Imaging and Communications in Medicine (DICOM) standard, and data formatted according to this standard may be said to be in a DICOM format. Data stored in the DICOM format may for example include images, such as medical scan images, reports, text, and information entered into particular data fields, such as information relating to the subject. Data stored according to the DICOM standard includes DICOM tags, which may be standardized data fields used to reference data included in a medical imaging study. Medical imaging studies as discussed herein may, in some embodiments, comprise data formatted according to the Digital Imaging and Communications in Medicine standard.

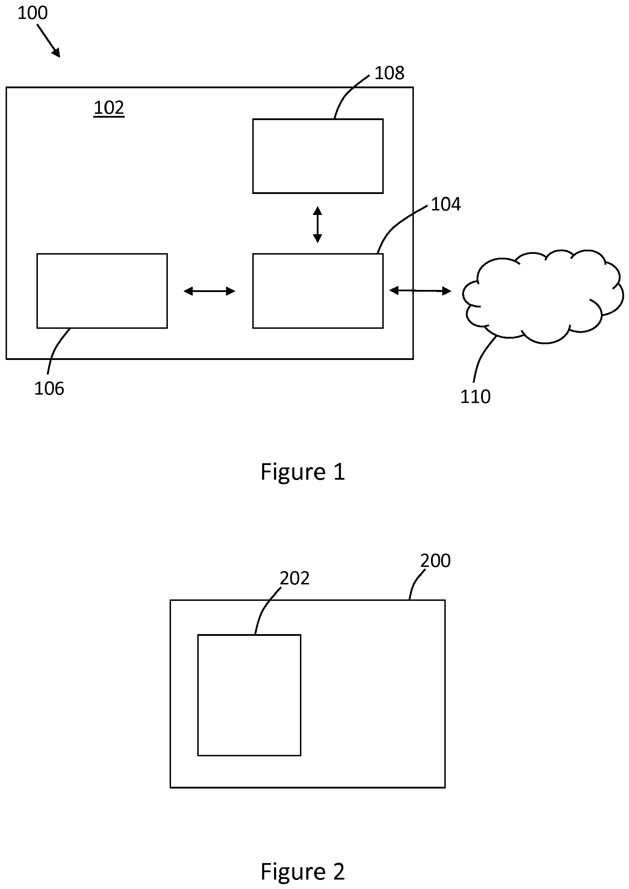

Referring to the drawings, Figure 1 shows, schematically, an example of a system 100 for storing data, such as medical imaging data stored in the DICOM format. The system 100 includes a network 102 which, in this example, is a clinical network. For example, the network 102 may be a computer network comprising servers and/or computing devices distributed throughout a network of medical facilities, such as hospitals. The network 102 includes an apparatus 104, such as a computing device or system which is capable of handling and manipulating data, and controlling movement of data between computing devices and storage media. In some examples, the apparatus 104 may comprise or form a part of a picture archiving and communication system (PACS), which is a system used for storing and sharing data, such as medical imaging data, amongst various entities, such as medical facilities.

The network 102 also includes an imaging system 106 which may be used to acquire imaging data. The imaging system 106 may comprise a system configured to capture imaging data in one of a number of imaging modalities. For example, the imaging system 106 may comprise a computed tomography (CT) scanning device, a magnetic resonance (MR) device or a computed axial tomography (CAT) scanning device. Other imaging modalities may alternatively by used to capturing imaging data. The imaging system 106 is in communication with the apparatus 104 such that data may be exchanged between the imaging system and the apparatus. For example, data acquired by the imaging system 106 may be transmitted to the apparatus 104 for processing.

The network 102 also includes a first storage medium 108 which is, in this example, in communication with the apparatus 104. The apparatus 104 may deliver data to the first storage medium 108 for storage, and may communicate with the first storage medium to retrieve data from storage. The first storage medium 108 may be considered to be a secure "on-site" storage medium. In other words, the first storage medium 108 may be located within the network 102 and may be accessible only by authorized people using devices connected to or within the network, and/or who have access to the network.

The system 100 also includes a second storage medium 110, located outside the network 102. The second storage medium 110 may be considered to be an "off-site" storage medium. In some examples, the second storage medium 110 may be a cloud-based storage medium. Such cloud-based storage facilities may be made available by cloud-computing service providers, and may, for example, provide a data storage facility in exchange for a fee. Data may be transmitted to, and retrieved from, the second storage medium 110 by the apparatus 104. In some examples, the second storage medium 110 may be considered to be less secure than the first storage medium 108 as there may be a greater chance that data may be obtained from the second storage medium, for example, during a security breach.

According to a first aspect of the invention, an apparatus is provided for storing medical imaging data. Figure 2 shows a schematic illustration of an example of an apparatus 200 for storing medical imaging data. The apparatus 200 may comprise, or be functionally equivalent or similar to, the apparatus 104 discussed above. The apparatus 200 comprises a processor 202, which is configured to acquire a medical imaging study relating to a subject. The medical imaging study may be acquired, for example, from the imaging system 106, or from a storage medium storing previously-acquired imaging studies. In some embodiments, the medical imaging study may be provided to the processor 202 manually, for example by a user uploading one or more files containing medical imaging studies.

The processor 202 is further configured to identify elements in the medical imaging study that are indicative of the identity of the subject. As noted above, the medical imaging study may be in accordance with the DICOM standard, and may include image data, such as a series of CT or MR scans, and textual data, such as information relating to the subject, details of the imaging modality used to capture the scans, and details of a medical professional's interpretation of the scan or a diagnosis following a review of the scan. Some elements in the study may identify the subject. For example, the study may include the subject's name, address and date of birth as textual entries. In some examples, the study may include an image or a scan showing the subject's face, from which the subject may be identified. Any elements which might be indicative of the identity of the subject are identified by the processor 202.

Various methods may be used to identify image features in the image which are indicative of the identity of the subject. For example, identifying such subject-identifying image features may comprise applying at least one of: an atlas-based segmentation technique; a sliding-window detection technique; a generalized Hough transform voting technique; a regression forest voting technique; an image segmentation technique; and a deep-learning technique.

Atlas-based segmentation, or registration, involves identifying features of an image which correspond to (i.e. look the same as) known features. Features, such as anatomical features (e.g. skin markings or body parts) or facial features (e.g. the eyes, the nose, the mouth, the ears and the mandible), may be labelled in one or more training images, for example using a statistical atlas of a human body and, from those labels, the same or similar features may be identified in new images. A spatial transformation may be estimated between an atlas (reference) image and a target image, in order to identify relevant regions in the image for anonymization. Sliding window detection uses a patch-wise analysis of the image (in a sliding window fashion). For each patch, a set of features is computed which are used by a classification algorithm to determine whether or not the patch should be subject to anonymization. Generalized Hough Transform and regression forest voting both allow for a patch-wise prediction of the location of organs or landmarks (e.g. for anonymization purposes). Deep learning is a special instance of image segmentation, using fully convolutional neural networks. Deep learning techniques use artificial neural networks consisting of a sequence of interconnected layers (e.g. a convolutional layer, a max-pooling layer, an up-convolutional layer), producing a pixel-wise segmentation of an input image.

The processor 202 is further configured to anonymize the medical imaging study by removing the identified elements from the medical imaging study. By removing those elements of the medical imaging study which might be indicative of the subject's identity, the medical imaging study becomes anonymized, in that it will not be possible to identify the subject from the data remaining in the study. Various methods of anonymizing the study may be used, as discussed herein. In general, however, textual data in the medical imaging study may be anonymized by removing any subject-identifying data, for example data entered under particular DICOM tags, from the study. Image data, such as scans in included in the medical imaging study may be anonymized by removing those portions of the image data which might be used to identify the subject. For example, if the image data comprises an image of the subject's face, the subject's facial features, such as the eyes, nose, ears and mouth, may be identified as potentially indicating the identity of the subject, and these identified features may be removed from the image data in the study. Removal of the identified elements may include complete removal, to leave an empty space or to set the identified elements to a defined value (e.g. include a solid block of color), covering or masking the identified elements such that the identified elements cannot be viewed or obtained, or obscuring the identified elements in some way, for example, using blurring or pixelating techniques.

It is intended that, once the medical imaging study has been anonymized, the study does not include any data which can be used to identify the subject to whom the study relates. It will be appreciated that some of the data remaining in the anonymized study may provide some indication of the likely identity of the subject, even if it cannot be used to determine the exact identity of the subject. For example, a scan may show an outline of the subject's head. If the head shape is distinctive to the subject, then this may provide an indication of the subject's identity. Thus, the processor 202 may be configured to remove a minimum defined set of data from the medical imaging study, such as a defined set of DICOM data elements most likely to contain subject-identifying data, or image data relating to a defined set of features (e.g. facial features) of the subject. In this way, the likelihood that the subject's identity can be determined from the anonymized study may be negligible.

The processor 202 is further configured to deliver the anonymized medical imaging study for storage at a first location. Since the anonymized study contains no data from which the subject's identity can be determined, the anonymized study may be stored in a relatively less secure storage medium, as it would not be possible to determine the subject's identity even if the data were intercepted or accessed, for example, in the event of a security breach in the storage facility. In some embodiments, the first location may comprise the off-site storage medium 110 discussed above. The first location may, for example, comprise a cloud-based storage medium.

The processor 202 is further configured to deliver data relating to the identified elements for storage at a second location. Thus, the data which might indicate the identity of the subject is delivered for storage at the second location. In some embodiments, the second location may be different from the first location. In other embodiments, the second location may be the same as the first location. The second location may comprise a more secure storage medium than the first location. The storage at the second location may, in some embodiments, comprise the on-site storage medium 108 discussed above. In some embodiments, the second location may comprise a cloud-based storage medium but, preferably, high-security storage medium. In this way, the identified data can be stored in a relatively more secure storage medium than the remaining data (i.e. the anonymized medical imaging study) from which the subject-identifying data has been removed.

As noted above, the medical imaging study may include image data (e.g. a scan image) and non-image data (e.g. textual data). According to some embodiments, the medical imaging study may comprise at least an image. The identified elements of the medical imaging study (i.e. those elements identified as being indicative of the identity of the subject) comprise image features in the image which are indicative of the identity of the subject. In such embodiments, anonymizing the medical imaging study may comprise removing the identified image features from the image. The processor 202 may be configured to deliver the anonymized image for storage as part of the anonymized medical imaging study at the first location. Thus, the image may have any subject-identifying features removed (e.g. blanked, covered, blurred or obscured), and the anonymized image may then be stored at the first location (e.g. in the off-site storage medium 110). The processor 202 may be further configured to deliver data relating to the identified image features for storage at the second location. Thus, data relating to those image features from which the subject may be identified may be stored at the second location (e.g. in the on-site storage medium 108), which may, in some embodiments, be more secure.

In some embodiments, an image in the medical imaging study may include annotations, such as notes relating to the image, which may also include information from which the subject may be identified. Such annotations may also be examined for information which might identify the subject. If it is determined that any annotations contain text (e.g. handwritten notes) which could be used to identify the subject, then these may also be removed from the study and delivered for storage at the second location. Such annotations may be converted to text using optical character recognition (OCR) techniques, then analyzed as text in a manner similar to the non-image data included in the study. Alternatively, or additionally, an annotation may be treated as an image feature, and annotations identified as being indicative of the subject's identity may be removed as an image feature, using methods discussed herein.

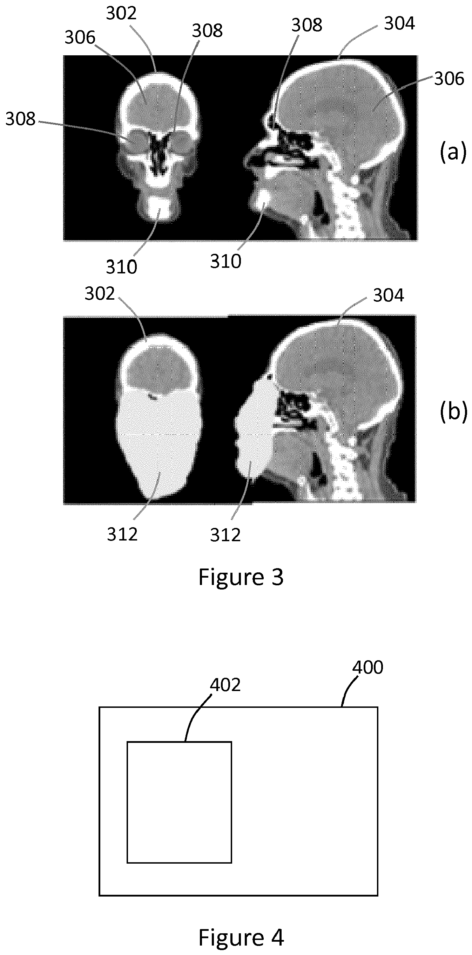

Figure 3 shows an example of an image that might be included in a medical imaging study. Figure 3a shows a front scan 302 and a side scan 304 of a subject's head in the form in which it may be acquired captured by the imaging system 106. The processor 202 may be configured to identify particular features in the scan, such as a brain 306, eyes (or eye sockets) 308, and a/or a mandible 310. In other embodiments, other features (e.g. the ears) may be identified. In general, the image features in the image which are indicative of the identity of the subject may comprise at least one of: an anatomical feature, an annotation, an implant, and a tattoo. While, in the examples discussed herein, the features are facial features, in other examples in which parts of a body other than the head are scanned, other features of the subject's body may be identified, for example using feature recognition techniques.

In the example of Figure 3, the eyes 308 and the mandible 310 may be indicative of the identity of the subject and, therefore, the processor 202 may be configured to remove the eyes and the mandible from the scan image, as shown in Figure 3b. In some embodiments, such as the embodiment shown in Figure 3b, the processor 202 may be configured to replace the removed identified image features with a mask 312 in the anonymized medical imaging study. Thus, the identified features have been removed and replaced with the mask 312, referred to as an anonymization mask. The brain 304 and other features of the scan are still visible in the slice 302 and the slice 304 of Figure 3b, but the mask 312 replaces the identified features (i.e. the eyes 308 and the mandible 310 in this example) so that the remaining scan images do not contain sufficient data from which the identity of the subject can be revealed.

In the embodiment shown in Figure 3, an anonymization mask is used to replace the features removed from the image. A mask may be useful to indicate the portion of the image from which data has been removed. Thus, it can easily be seen that the masked portion of the image is the portion which contained subject-identifying image features. In other embodiments, the image features may be removed by encrypting, obscuring or hiding the features using a particular algorithm or encryption key which is known only by the processor removing the features, and which can be reversed only by the same processor or by an authorized processor. In other words, the identified image features may be disguised and the original image can only be obtained if the disguise can be removed or reversed.

Since portions of the medical imaging study may be stored in different locations, it is important to be able to locate the various portions of a study in the different locations when it is intended to retrieve the study from storage. Thus, in some embodiments, the processor may be configured to assign a first identifier to the anonymized medical imaging study and assign a second identifier to the data relating to the identified elements. The first identifier and the second identifier may correspond to one another. In this way, a processor may be able to retrieve the various portions of the study from the various storage locations, and match them to one another. For example, an anonymized medical image study may be assigned an identifier in the form of the reference '1234a'. The data relating to the identified elements may be assigned a corresponding identifier in the form of the reference '1234b'. Thus, the processor retrieving the study knows, or can determine, from the numerical part of the reference (1234) that the two parts of the study are related to one another, and the labels 'a' and 'b' are used to distinguish the parts of the study from one another. In some embodiments, the identifiers assigned to the various portions of the study may be unique identifiers to further improve security.

The storage of the various portions of the medical imaging study may be made more secure by employing known encryption techniques. For example, the processor 202 may be further configured to encrypt at least one of: data relating to the anonymized medical imaging study, and the data relating to the identified elements. In some embodiments, a more secure encryption method may be used for the data relating to the identified elements, to reduce the likelihood that those elements, which may be used to identify the subject, can be accessed. In some embodiments, the anonymized medical imaging study and the data relating to the identified elements may both be stored in a cloud-based storage medium. In such embodiments, the data relating to the identified elements may be encrypted using a secure encryption method, and/or may be stored in a more secure cloud-based storage environment than the anonymized medical imaging study.

In some embodiments, both the sensitive data in the medical imaging study (i.e. elements that are indicative of the identity of the subject) and the less sensitive data (the anonymized study) may be stored in the same storage facility (e.g. if the first location is the same as the second location). In such embodiments, the sensitive data may be provided with a higher level of security. In some embodiments, a key, or indicator, linking the sensitive data and the less sensitive data in a particular study may be stored in a secure storage facility, for example in the first storage medium 108, or elsewhere in the network 102, such that only an authorized person or computing device is able to access the linking key. In this way, even if the anonymized study and the subject-identifying features are accessed separately, they cannot be combined without also having access to the linking key.

According to some embodiments, the medical imaging study may comprise at least non-image data. In this context, non-image data may be considered to be textual data. For example, as discussed above, non-image data may include textual data entered into various data fields, and/or entered with reference to one or more DICOM tags. The processor 202 may be further configured to identify a data element in the non-image data that is indicative of the identity of the subject. Such a data element may be identified, for example, by identifying particular DICOM tags in the study which may relate to data from which the subject may be identified, such as the subject's name or date of birth. In some embodiments, identifying a data element in the non-image data may comprise at least one of: identifying a data element in the non-image data that is present in a defined list of data elements known to be indicative of the identity of the subject; and identifying a data element in the non-image data using a text search engine. In other words, the processor 202 may be configured to search through any text included in the medical imaging study to identify any words, terms or phrases included in a defined list. For example, the defined list may include the subject's name, address, and data of birth, and the processor 202 may search for these details in the study. Alternatively, or additionally, a search engine, or search mechanism may be employed to search the non-image data for a character, word, term and/or phrase entered as a search query.

The processor 202 may be further configured to anonymize the non-image data by removing the identified data element from the non-image data. Non-image data may be removed and included within a separate data file, for example.

The processor 202 may be further configured to deliver the anonymized non-image data for storage at the first location. Thus, the anonymized non-image data may be stored along with the anonymized image data in a storage medium at the first location. In some embodiments, the anonymized image data and the anonymized non-image data may be stored in an anonymized version of the medical image study at the first location.

The processor 202 may be further configured to deliver the remaining non-image data for storage at the second location. The remaining non-image data may include any non-image data (e.g. textual data) which is not considered to be indicative of the identity of the subject and, therefore, need not be stored in a secure storage environment. The remaining non-image data may be stored along with the identified image features in a storage medium at the second location. As noted above, the storage medium at the first location may be relatively more secure than the storage medium at the second location, such that the image and non-image data which could be used to identify the subject is stored in a more secure storage environment than the data which cannot be used to reveal the identity of the subject.

So far, embodiments of the invention have been described in terms of the storage of the various portions of a medical imaging study. Aspects of the invention also relate to retrieving data from storage.

Figure 4 shows a schematic illustration of an example of an apparatus 400 for retrieving from storage medical imaging data associated with a subject. The medical imaging data may, for example, be data stored by the apparatus 200 discussed herein. In some embodiments, the apparatus 400 may be the same as the apparatus 200. In other words, the apparatus 200 may be configured to store medical imaging data, and retrieve the medical imaging data from storage. The apparatus 400 comprises a processor 402 configured to obtain, from a first storage location, an anonymized medical imaging study associated with the subject, the anonymized medical imaging study having had elements indicative of the identity of the subject removed therefrom.

The processor 402 is further configured to obtain, from a second storage location, data relating to the elements indicative of the identity of the subject. The medical imaging study may comprise both image data and non-image data and, therefore, the elements indicative of the subject's identity may comprise both image data elements and non-image data elements, such as textual data.

The processor 402 is further configured to form a medical imaging study by combining the anonymized medical imaging study and the data relating to the elements indicative of the identity of the subject. In some embodiments, the anonymized medical imaging study and the data relating to the elements indicative of the identity of the subject may have associated identifiers assigned thereto, so that the obtained anonymized study can be combined with the correct corresponding data relating to the subject-identifying elements. Combining the various portions to form the medical imaging study may be achieved by incorporating the removed data (i.e. the data relating to the elements indicative of the identity of the subject) into the anonymized medical imaging study in the positions from which the data was removed. Each item of removed data may, for example, be stored with a location indicator, indicating the location in the medical imaging study from which it was removed.

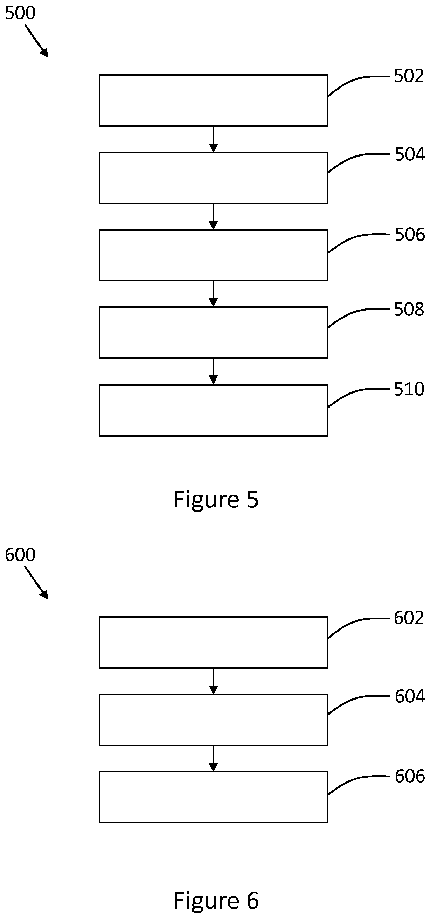

A further aspect of the invention relates to a method for storing medical imaging data. Figure 5 is a flowchart of an example of a method 500 for storing medical imaging data according to embodiments of the invention. The method 500 comprises, at step 502, acquiring a medical imaging study relating to a subject. The medical imaging study may, for example, be acquired from the imaging system 106, or from a storage medium. At step 504, the method 500 comprises identifying elements in the medical imaging study that are indicative of the identity of the subject. The identified elements may comprise image data elements and/or non-image data elements. The method 500 comprises, at step 506, anonymizing the medical imaging study by removing the identified elements from the medical imaging study. At step 508, the method 500 comprises delivering the anonymized medical imaging study for storage at a first location. At step 510, the method 500 comprises delivering data relating to the identified elements for storage at a second location. In some embodiments, the delivering steps 508 and 510 may be combined into a single step.

A further aspect of the invention relates to a method for retrieving medical imaging data from storage. Figure 6 is a flowchart of an example of a method 600 for retrieving from storage medical imaging data associated with a subject. The method 600 comprises, at step 602, obtaining, from a first storage location, an anonymized medical imaging study associated with the subject, the anonymized medical imaging study having had elements indicative of the identity of the subject removed therefrom. At step 604, the method 600 comprises obtaining, from a second storage location, data relating to the elements indicative of the identity of the subject. The method 600 comprises, at step 606, forming a medical imaging study by combining the anonymized medical imaging study and the data relating to the elements indicative of the identity of the subject.

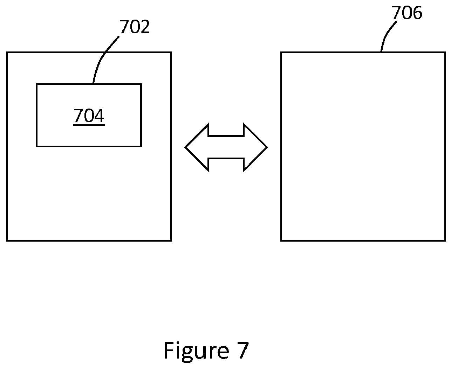

A further aspect of the invention relates to a computer program product. Figure 7 shows, schematically, a computer-readable medium and a processor. According to some embodiments, a computer program product comprises a non-transitory computer readable medium 702, the computer readable medium having computer readable code 704 embodied therein, the computer readable code being configured such that, on execution by a suitable computer or processor 706, the computer or processor is caused to perform a method as described herein.

The processor 202, 402, 706 can comprise one or more processors, processing units, multi-core processors or modules that are configured or programmed to control the apparatus 104, 200, 400 in the manner described herein. In particular implementations, the processor 202, 402, 706 can comprise a plurality of software and/or hardware modules that are each configured to perform, or are for performing, individual or multiple steps of the method described herein.

It will be appreciated that the embodiments of the invention also apply to computer programs, particularly computer programs on or in a carrier, adapted to put embodiments of the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to an embodiment of the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

While the invention has been illustrated and described in detail in the drawings and in the foregoing description, such illustration and description are to be considered illustrative and exemplary, not restrictive. The invention is not limited to the disclosed embodiments. Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for storing medical imaging data, the apparatus comprising a processor (202) configured to:
acquire a medical imaging study relating to a subject;
identify elements in the medical imaging study that are indicative of the identity of the subject;
anonymize the medical imaging study by removing the identified elements from the medical imaging study;
deliver the anonymized medical imaging study for storage at a first location; and
deliver data relating to the identified elements for storage at a second location.

2. The apparatus (200) of claim 1, wherein the medical imaging study comprises at least an image;
wherein the identified elements of the medical imaging study comprise image features in the image which are indicative of the identity of the subject;
wherein anonymizing the medical imaging study comprises removing the identified image features from the image; and
wherein the processor is configured to:
deliver the anonymized image for storage as part of the anonymized medical imaging study at the first location; and
deliver data relating to the identified image features for storage at the second location.

3. The apparatus (200) of claim 1 or claim 2, wherein the processor (202) is further configured to:
assign a first identifier to the anonymized medical imaging study; and
assign a second identifier to the data relating to the identified elements;
wherein the first identifier and the second identifier correspond to one another.

4. The apparatus (200) of claim 2 or claim 3, wherein the processor (202) is further configured to:
replace the removed identified image features with a mask in the anonymized medical imaging study.

5. The apparatus (200) of any of claims 2 to 4, wherein identifying image features in the image which are indicative of the identity of the subject comprises applying at least one of: an atlas-based segmentation technique; a sliding-window detection technique; a generalized Hough transform voting technique; a regression forest voting technique; an image segmentation technique; and a deep-learning technique.

6. The apparatus (200) of any of claims 2 to 5, wherein the image features in the image which are indicative of the identity of the subject comprise at least one of: an anatomical feature, an annotation, an implant, and a tattoo.

7. The apparatus (200) of any of the preceding claims, wherein the processor (202) is further configured to:
encrypt at least one of: data relating to the anonymized medical imaging study, and the data relating to the identified elements.

8. The apparatus (200) of any of the preceding claims, wherein the medical imaging study comprises at least non-image data, and wherein the processor (202) is further configured to:
identify a data element in the non-image data that is indicative of the identity of the subject;
anonymize the non-image data by removing the identified data element from the non-image data;
deliver the anonymized non-image data for storage at the first location; and
deliver the remaining non-image data for storage at the second location.

9. The apparatus (200) of claim 8, wherein identifying a data element in the non-image data comprises at least one of:
identifying a data element in the non-image data that is present in a defined list of data elements known to be indicative of the identity of the subject; and
identifying a data element in the non-image data using a text search engine.

10. An apparatus for retrieving from storage medical imaging data associated with a subject, the apparatus comprising a processor (402) configured to:
obtain, from a first storage location, an anonymized medical imaging study associated with the subject, the anonymized medical imaging study having had elements indicative of the identity of the subject removed therefrom;
obtain, from a second storage location, data relating to the elements indicative of the identity of the subject; and
form a medical imaging study by combining the anonymized medical imaging study and the data relating to the elements indicative of the identity of the subject.

11. The apparatus (400) of any of the preceding claims, wherein the second location comprises a more secure storage medium than the first location.

12. The apparatus (400) of any of the preceding claims, wherein the medical imaging study comprises data formatted according to the Digital Imaging and Communications in Medicine, DICOM, standard.

13. A method for storing medical imaging data, comprising:
acquiring (502) a medical imaging study relating to a subject;
identifying (504) elements in the medical imaging study that are indicative of the identity of the subject;
anonymizing (506) the medical imaging study by removing the identified elements from the medical imaging study;
delivering (508) the anonymized medical imaging study for storage at a first location; and
delivering (510) data relating to the identified elements for storage at a second location.

14. A method for retrieving from storage medical imaging data associated with a subject, the method comprising:
obtaining (602), from a first storage location, an anonymized medical imaging study associated with the subject, the anonymized medical imaging study having had elements indicative of the identity of the subject removed therefrom;
obtaining (604), from a second storage location, data relating to the elements indicative of the identity of the subject; and
forming (606) a medical imaging study by combining the anonymized medical imaging study and the data relating to the elements indicative of the identity of the subject.

15. A computer program product comprising a non-transitory computer readable medium, the computer readable medium (702) having computer readable code (704) embodied therein, the computer readable code being configured such that, on execution by a suitable computer or processor (706), the computer or processor is caused to perform the method of any claims 13 or 14.
